# EUROPEAN PATENT SPECIFICATION

(11) **EP 0 597 878 B1**
(45) Date of publication and mention of the grant of the patent: **27.08.1997**
(21) Application number: 92914187.7
(22) Date of filing: 29.01.1992
(51) Int. Cl.: A61B 5/00

(54) **ELECTROMAGNETIC METHOD AND APPARATUS TO MEASURE CONSTITUENTS OF HUMAN OR ANIMAL TISSUE**
VERFAHREN UND VORRICHTUNG ZUR ELEKTROMAGNETISCHEN BESTIMMUNG VON IM LEBENDEN GEWEBE VORLIEGENDEN SUBSTANZEN
METHODE ET APPAREIL ELECTROMAGNETIQUE PERMETTANT DE MESURER LES CONSTITUANTS DE TISSUS HUMAINS OU D'ANIMAUX

(30) Priority: 03.07.1991 US 725441
(43) Date of publication of application: 25.05.1994
(73) Proprietor: VIVASCAN CORPORATION, Southboro, MA 01772 (US)
(72) Inventor: HARJUNMAA, Hannu, Holden, MA 01520 (US); MENDELSON, Yitzhak, Worcester, MA 01609 (US); WANG, Yi, Worcester, MA 01609 (US)
(74) Representative: Greenwood, John David
(86) International application number: US9200774
(87) International publication number: WO9300855

(56) References cited:
- EP-A- 0 003 015
- EP-A- 0 140 633
- EP-A- 0 160 768
- EP-A- 0 401 453
- WO-A-89/11825
- WO-A-91/15992

## Description

### Background of the Invention

This invention relates to the non-invasive measurement of the concentration of substances that absorb electromagnetic radiation, such as light or infrared radiation, in absorbing and turbid matrices, such as human or animal body tissue, using a probe beam of electromagnetic radiation. The invention is described as applied to the special cane of glucose measurement in human tissue using near-infrared radiation. It is, however, generally applicable to measurements of the concentration of any species that absorbs electromagnetic radiation, especially in strongly absorbing and turbid matrices.

The infrared measurement methods known in the art are not well adapted to the problem of quantifying an analyte dissolved in a strongly absorbing solvent. The known methods include separate or directly alternating measurements at a "glucose" wavelength and at a "reference" wavelength, where glucose does not absorb, as well as differential wavelength modulation about a glucose absorption band (C. Dähne, D. Gross, European Patent 0 160 768 and references therein). In the known methods, the signal is easily lost into the variable and strong background presented by water and other constituents in the tissue and in the capillary blood flow.

### Summary of the Invention

The present invention is an improvement over European Patent EP-A-0 401 453 (Harjunmaa), referenced above. In Harjunmaa, a balanced differential modulation method is disclosed wherein a radiation beam comprised of alternating pulses of two wavelengths forming a combined beam, is balanced or nulled using a reference detector that takes a sample of the combined beam before it enters the tissue and is detected by a primary detector. Although suitable for the purposes intended, the precautions taken to deal with the unavoidable differences in the spectral response between the reference detector and the primary detector make the system somewhat complicated.

The balanced differential (or balanced bridge) method of Harjunmaa utilizes two wavelengths that have the special property of having identical extinction coefficients in the sample matrix. A radiation beam is generated that contains these two wavelengths in alternate succession at a suitable frequency. When the beam is properly balanced for the measurement, a detector placed to detect radiation transmitted or reflected by the sample does not detect any alternating component in the radiation; when the sample is inserted into the beam path, the same detector also would detect no alternating component, if the matrix do not contain any of the analytes. Only in the case where there is some analyte in the sample matrix will the detector detect an alternating signal synchronous to the wavelength alternation. This feeble alternating signal is amplified and is then detected using a phase-sensitive detector (or lock-in amplifier).

In the method and apparatus of the present invention, the concentration measurement is accomplished using a two-wavelength alternating radiation probe beam which interacts with the tissue. One of the wavelengths is tunable to account for the expected variability of the background spectrum. Detected signals from the probe beam after passing through the matrix are balanced or nulled with a given unknown reference concentration of analyte present by tuning the variable wavelength beam over a range of frequencies. Next, the fluid balance of the tissue in the radiation beam is changed thereby changing the ratio of the analyte concentration to the reference concentration. The alternating component of the interacted probe beam is then detected. The amplitude of the alternating-current (AC) signal given by the detector represents analyte concentration or the difference from a preset reference analyte concentration. The interaction of radiation with tissue can occur in either a reflecting or transmissive mode.

### Brief Description of the Drawings

Fig. 1 is a block diagram of the apparatus of the invention.

Fig. 2 is a side view of a measuring head for use with the apparatus of Fig. 1 in a reflective mode of operation.

Fig. 3 is a sectional view taken along lines III-III of Fig. 2.

Fig. 4 is a sectional view taken along lines IV-IV of Fig. 2.

### Detailed Description of the Invention

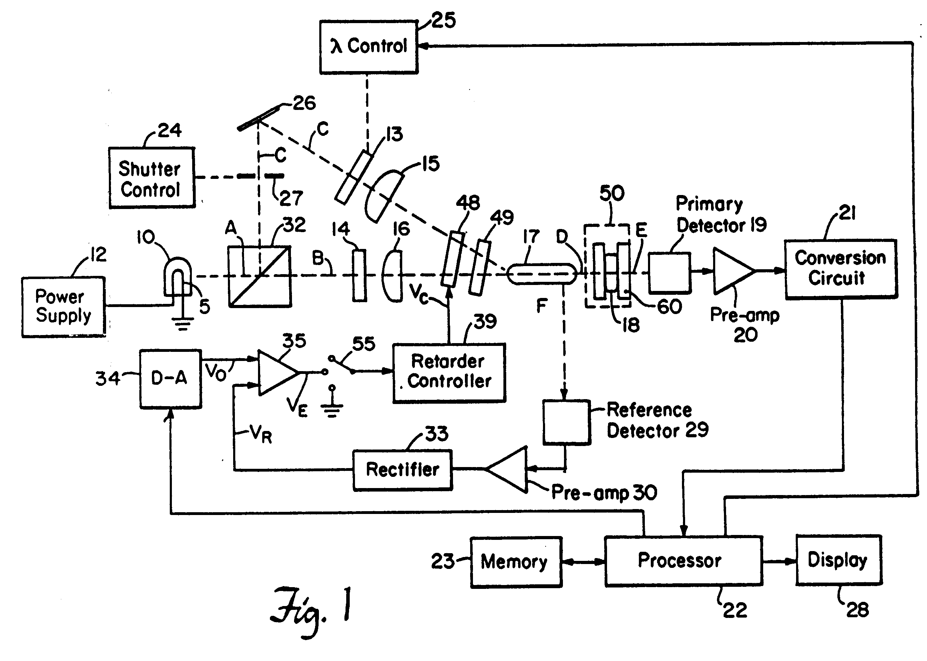

Referring now to Fig. 1 the invention will now be described in detail in connection therewith.

A light source 10 generates a beam A of electromagnetic energy or radiation preferably in the infrared range of the light spectrum. Preferably, the intensity of the beam A is maintained constant by power supply 12 coupled to lamp filament 5. Beam A is split into two beams, B and C, of equal intensity using polarizing beam splitter 32. Beam C is reflected by mirror 26. Both beams B & C are directed through respective interference filters 14 and 13 and focusing lenses 16 and 15, toward optical beam combiner 17.

Filters 14 and 13 are used to select the preferred wavelengths for beams B and C respectively. In the present system, the wavelength λ₁ for the constant wavelength beam B is at 1600 nm while the center wavelength λ₂ of the variable wavelength beam C is at 1750 nm. The wavelength of beam C is varied by control 25 which mechanically tilts filter 13 to move the wavelength λ₂ of the beam C to shorter wavelengths.

These wavelengths are specifically selected for glucose concentration determination since glucose absorbs at 1600 nm and glucose absorption is substantially less and fairly constant in the range of 1670-1730 nm.

To select one of the wavelengths at a time, the two constituent beams B and C are directed at a liquid crystal variable retarder 48. It is controlled electrically, by a control voltage Vc from controller 39, to alternate between two states: nonrotating and rotating. After the retarder the beams encounter a sheet polarizer 49. Its function is to transmit only one linear polarization component. The rotator will either do nothing or rotate both constituent beam polarizations about 90 degrees. Thus, either one or the other constituent beam will be transmitted, alternating at the amplitude modulation frequency of the AC controlling voltage Vc. The retarder 48 also controls the intensity relation of the two wavelength half-periods by producing elliptically polarized intermediate states, according to the control voltage Vc, and thereby causing reduced transmittance of the given constituent beam through the polarizer 49. In that case, there will be a corresponding residual transmittance for the other constituent beam. This residual transmittance has no effect on the operation of the system since only the AC component of the beam intensity is detected.

The two beams B and C are combined in a beam combiner 17 which may comprise a tapered aluminum tube having a polished interior surface. A glass rod could also be used, as well as an integrating sphere or film optics. A portion F of the combined beam is sampled by a reference detector 29 through a hole drilled in the side of the beam combiner 17.

A main portion of the combined beam D enters a sampling area 18 of the body, such as a fingerweb or an earlobe and is attenuated by the body tissue. The resultant beam E is detected by a photoconductive lead sulfide (PbS) infrared primary detector 19 operating at room temperature. It is important that the two detectors 19 and 29 be closely similar in properties to minimize the offset between them that exists even when they are detecting the same beam. Their spectral sensitivity peaks at about 2.2...2.5 micrometers. The PbS detectors are operated in bolometer circuits, AC-coupled to preamplifiers 20 and 30. Any other detector sensitive in the relevant wavelength range could be used, with the appropriate coupling and amplifying method.

The primary detector preamplifier 20 is connected to a conversion circuit 21 that phase-sensitively rectifies the analog signals produced by the preamplifier and converts them into digital form. The conversion circuit is connected to a computer processor 22. The processor has at its disposal a memory 23 and a display unit 28.

The reference detector preamplifier is connected to a phase-sensitive rectifier circuit 33 which generates a signal V_{R} indicative of the difference between the intensities of beams B and C. There is also provided a D/A-converter 34 in which the processor 22 sets the previously recorded offset analog voltage value Vo between the two detectors for the current wavelength. The voltage outputs of the phase-sensitive rectifier and of the D/A-converter are fed to a difference amplifier 35 that provides an error signal V_{E} to the variable retarder controller 39.

In this example, the measurement is performed on the fingerweb. For that purpose there is provided an adjustable gap mechanism 60 where the fingerweb is inserted for measurement. A temperature control mechanism 50 maintains the sample temperature uniform at body temperature during measurement.

A shutter mechanism 27 is provided to interrupt one of the constituent beams for intensity calibration purposes as explained below.

A glucose determination consists of three steps, called the equalizing, balancing and measuring steps:

An equalizing step is performed preferably before every measurement. During equalization, the sample 18 is not in the beam path. For equalization to begin, the filter 13 is tilted to one extreme of its wavelength range by control mechanism 25. If there is any AC signal output from the primary detector the output of D/A-converter 34 is changed by processor 22 which in turn causes controller 39 to generate a control voltage Vc which varies the intensity relation between the two alternating beams C and B until a zero primary detector signal output is obtained. The wavelength tuning range is now scanned from end to end by tilting filter 13. At suitable intervals, the offset value Vo required at D/A-converter 34 to bring the output of the primary detector 19 to zero is recorded in memory. this completes the equalization step.

To perform the balancing step, the sample area 18 or fingerweb is introduced between the beam D and the detector 19 in the adjustable gap mechanism 60. The mechyanism 60 gently squeezes the web, reducing the thickness of the tissue in the optical path. To avoid hurting the subject, there is a preset maximum pressure at which the squeezing stops even if the target thickness has not been reached. The wavelength tuning range is again scanned, until the alternating signal in the primary detector 19 vanishes. Continuously during this operation, the processor, as it changes the wavelength, also updates the offset voltage in the D/A-converter 34 to reproduce the condition that, in the equalizing step, gave a zero primary detector signal output. When the balancing wavelength is found, scanning is stopped, but the reference detector 29 continues to control the intensity relation and will do so throughout the measuring step.

As the wavelength is changed, the sensitivity of the system to glucose will also change slightly. This is compensated for by reading the correct sensitivity coefficient from a look-up table in the memory. The coefficients are obtained through previous calibration at each wavelength (as described below).

On the basis of the known properties of the instrument, it is known approximately how large the single-wavelength signal amplitude is at a given sample thickness. This information is needed to obtain a quantitatively correct glucose concentration reading, since the alternating signal amplitude obviously scales with the said amplitude. to improve the accuracy of the result, the closed-loop control is disabled by grounding switch 55 so that the output of controller 39 does not change. One constituent beam C is blocked with shutter mechanism 24 and the amplitude of the transmitted single-wavelength beam B is measured, and used to normalize the alternating signal result by division.

To perform the measuring step, the adjustable gap meachanism 60, under processor command, now releases the squeeze or vise 60 on the finberweb 18, increasing the thickness of tissue in the optical path by a predetermined amount. The material added into the beam path is mostly blood. The optimum thickness increase depends on the wavelengths used. Preferably, it is equal to one attenuation length in the added tissue. One attenuation length is the length in which the power of the probe beam D is attenuated to 0.368 times its original value. Blood has a differential absorbance different from that of the background tissue, thus the signal at the primary detector 19 departs from zero as the tissue thickness is increased. The resultant primary detector output signal is proportional to the differential absorbance and thus to glucose concentration. The result is displayed in the display unit 28.

To improve the stability of the readings, the thickness change may be made cyclical at a frequency in the order of 1 to 10 Hz, after balancing is first accomplished with stationary squeezed tissue. Then the amplitude of the rectified signal form the primary detector represent the analyte concentration, and the eventual drift of the rectified signal in the squeezed state will have less effect on the result.

If during the balancing operation, the correct balance is not found due to the balancing wavelength being outside the wavelength tuning range, the processor displays a message declaring the sample out of range.

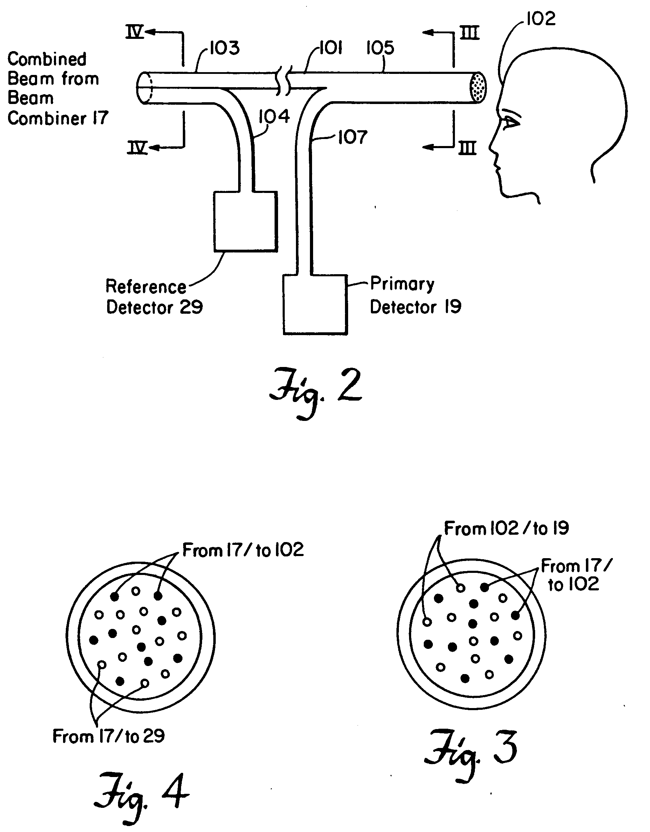

Figure 2A depicts a measuring head useful for operation in the reflective mode. The head 100 comprises a mixed optical fiber bundle 101 that is pressed against the forehead 102 of the subject for the balancing step and then released, while maintaining contact with the skin, to take the glucose reading. The combined beam D emerging from the beam combiner 17 is introduced into the fiber bundle transmitted leg 103. A bifurcation 104 diverts a portion of the infrared beam energy to the reference detector 29. For equalizing, the measuring end of the bundle 105 is pressed against a spectrally substantially neutral equalizing reflector (not shown) that diffuses a portion of the radiation into the receiving bifurcation 107 and on to the primary detector 19. The fibers in both common ends of the bundle are in a mixed arrangement as shown in the sectional views of Figs. 3 and 4 wherein some of the fibers F couple the light beam from 17 to detector 29 (shown in dark cross-section) and others couple the light beam from 17 to sample tissue on the forehead 102 of the subject or patient. There is no glass cover or window in the measuring end of the bundle; therefore direct reflection from the skin surface is excluded, because any path that leads from the transmitting fibers to the receiving fibers passes through the tissue. Direct reflection is unwanted, because the radiation reflected directly is much stronger than the radiation scattered inside the tissue, and would mask the weak signal obtained from subcutaneous tissue. For balancing, the measuring end is pressed against the forehead 102 and the correct reference wavelength is found. To change the fluid ratio in the forehead skin, the measuring end is slightly released and then the glucose reading is taken.

In the method of this invention, the correct reference wavelength, i.e. the one that has an extinction coefficient equal to that of the primary wavelength, is found by zeroing the AC signal with the tissue background included. The glucose measurement is achieved by modifying the extracellular/intracellular fluid ratio in the tissue and then taking an imbalance reading. If the fluid ratio change is brought about by changing the thickness of tissue between parts of the measuring head, the change of tissue thickness can be either positive or negative. A positive change, or a thickness increase, is preferred, because in that case the balancing can be done under conditions of a larger signal-to-noise ratio, as more transmitted power is available when the thickness is smaller. The thickness change can be made once or many times cyclically to improve signal-to-noise ratio by averaging.

The balancing done with the tissue sample already in the radiation beam cancels errors due to the skin surface and to the tissue in the radiation path, including errors caused by an unknown tissue temperature. No prior knowledge of the balancing wavelength is needed, as long as it is within the tuning range. A reference detector is provided to cancel errors due to variations in the beam intensity relation, which are chiefly due to changes in the lamp spectral output during the measurement. The balancing done in accordance with this invention will also cancel the errors due to the fact that measurements made at different times, although intended to be done at exactly the same measuring site are, in practice, done at slightly different sites. The method according to this invention is sensitive only to the variable component of the tissue as its fluid ratio is changed. The unavoidable differences between the spectral sensitivities of the principal detector and the reference detector, as well as the nonneutrality of the beam sampling for the reference detector, are compensated for by suing a special equalizing procedure.

As explained in Harjunmaa above, the signal obtained in a balanced differential measurement, when taken as a function of the path length in the sample, has a maximum value at a certain path length, which turns out to be the inverse of the extinction coefficient of the sample matrix, or one attenuation length. The extinction coefficient is the sum of the absorption and scattering coefficients. At wavelengths over 2000 nm, water absorbs so strongly that the optimum thickness is much less than 1 mm, a thickness difficult to obtain by squeezing any part of the human body without causing pain. The incremental balanced differential modulation method disclosed here makes it possible to obtain the maximal signal by letting the thickness increment be equal to the optimum path length, while at the same time using a total sample thickness more comfortable to the subject. Also, as the absorption coefficient may be different for different tests that have to be carried out at different wavelengths, it is easy to vary the thickness incremental accordingly.

It is to be noted that scattering by tissue is, at these wavelengths, comparable in magnitude to absorption as an extinction mechanism and causes the resultant extinction coefficient to be much larger than the water absorption coefficient alone.

The interfering substances present in the body may, even after a careful wavelength selection, produce a residual signal due to their differential absorbance. Because of this, a personal calibration step is required before this system is used for absolute glucose determination. The calibration is performed by taking a blood sample of the subject, determining its glucose content and at the same time performing a measurement according to this method. The signal obtained is recorded to correspond to the actual initial glucose concentration. The varying concentrations can then be later deduced by using the known sensitivity of this system to glucose obtained by measuring glucose calibration samples.

If it is judged necessary to improve the specificity of the method, more than one wavelength pair can be used. If, for example, two wavelength pairs are used, the measurement can be done in a sequential mode, where a complete measurement is made according to this disclosure, first using one wavelength pair, and then the wavelengths are changed and another measurement is made with those wavelengths. It is also possible, although it may lead to a more complicated apparatus, to multiplex more than two wavelengths into one measuring beam and then to extract the information pertaining to each wavelength pair from the multiplexed signal. Also, if more than one wavelength pair is used, at least one of the wavelengths may be common to more than one pair.

It is known that, whereas extracellular fluid, which includes interstitial fluid and blood, has a certain glucose concentration, intracellular fluid contains very little glucose, since the latter is consumed inside the cells. Changing the ratio of extracellular to intracellular fluid thus provides a means to modulate the tissue, or by artificial means, such as squeezing the tissue either between the transmitter and receiver parts of measuring head (in the transmission mode) or between a combined transmitter receiver and a suitable bone within the body, such as the forehead.

## Claims

1. Non-invasive method for measuring the concentration of predetermined analytes in living body tissue (18), comprising the steps of:
a) generating a combined beam (D) of electromagnetic radiation comprised of two alternate and repetitive periods of radiation (B, C) having different wavelengths, the two wavelengths having different absorption coefficients for the analyte being sought, at least one of the wavelengths (C) being tunable;
b) detecting the combined beam (D) with a primary detector (19) for generating a primary electrical signal proportional to the intensity of the combined beam (D);
c) detecting the combined beam (D) with a reference detector (29) for generating a reference signal proportional to the intensity of the combined beam (D) and wherein the electrical response generated by the two period radiations in both detectors (19, 29) is substantially equal in magnitude, thus producing a substantially zero alternating component in the primary electrical signal and the reference signal;
d) directing the combined beam (D) on the tissue (18) so that radiation transmitted or reflected by the tissue (18) reaches the primary detector (19);
e) controlling the intensity relation between the periods using the reference signal and tuning the wavelength (C) of one of the periods to obtain a substantially zero alternating component in the primary signal;
f) changing the ratio of extracellular fluid content to intracellular fluid content in the tissue (18); and
g) measuring the concentration of the analyte in the tissue (18) based on the change in the primary electrical signal as a result of the fluid ratio change.

2. The method according to Claim 1, where the combined beam (D) passes through the tissue (18) before being detected by the primary detector (19); and, optionally, wherein the step of changing the fluid ratio comprises changing tissue (18) thickness, for example either wherein the tissue (18) thickness change is substantially equal to the inverse of the extinction coefficient of the tissue (18) at the two wavelengths, or wherein the tissue (18) thickness change is repeated cyclically, and the primary electrical signal variation over the cycle is used as a measure of the concentration of the analyte in blood.

3. The method according to claim 1, wherein either:
(a) the step of changing the fluid ratio includes applying pressure on the surface of the tissue (18); or
(b) the fluid ratio change is caused by natural pulsation due to the heartbeat, and the step of measuring the concentration is synchronized to such pulsation; or
(c) the analyte is a homologous body material, and the wavelengths are within the range of 1 to 2.5 micrometers.

4. The method according to Claim 1 for measuring glucose concentration in human or animal body tissue (18) and having one of the wavelengths selected for the interval 2125-2185 nm and another one of the wavelengths from the interval 2240-2300 nm.

5. The method of claim 1 and either:
(a) wherein the analyte is glucose and one of the wavelengths is selected from the interval 1500-1650 nm and another one of the wavelengths is from the interval 1650-1800 nm; or
(b) where the step of measuring the concentration is repeated using one or more additional wavelength pairs and the desired result is obtained from the signal values at each wavelength pair using a mathematical transformation; or
(c) further comprising before the step of directing the combined beam (D) equalizing the primary detector (19) and reference detector (29) by controlling the intensity relation of the different wavelength (B, C) periods so as to continually produce a minimum alternating signal from the primary detector (19), tuning the tunable wavelength (C) over the tuning range and at the same time recording into a memory as a function of the tuned wavelength (C), a control signal required to produce the minimum alternating signal condition; or
(d) further comprising while the tissue (18) is in the combined beam (D), calibrating the response of the system by blanking one of a pair of wavelength periods and measuring the resulting transmitted alternating signal-wavelength signal amplitude and using the result to normalize the response of the system.

6. Apparatus for non-invasive measurement of the in vivo concentration of a predetermined analyte in bodily tissue (18);
a) radiation means (10) to generate an electromagnetic radiation probe beam (D) containing two alternating wavelength portions (B, C) where at least one of the wavelengths (C) is tunable and the intensity of the radiation during at least one wavelength portion is controllable;
b) optical means (17) to transmit the probe beam to the tissue;
c) reference detector means (29) to detect a representative portion (F) of the probe beam, prior to interaction with the tissue (18), and for generating a reference signal proportional to probe beam (D) intensity;
d) primary detector means (19) to detect at least a portion of the probe beam radiation (E) after interaction with the tissue (18) and to generate a primary signal proportional to interacted probe beam (E) intensity;
e) electrical means (34) to produce an off-set signal in response to the primary signal;
f) electrical means (35) to produce a control signal from the reference signal and the off-set signal;
g) control means (39) to control the intensity relation of the alternating portions (B, C) of the probe beams, according to the control signal;
h) changing means (60) to change the ratio of extracellular fluid content to intracellular fluid content in the tissue (18); and
i) computing means (22) to convert the change in the primary signal as a result of the fluid ratio change to concentration values of the analyte(s) sought.

7. Apparatus according to Claim 6, wherein the optical means (17) includes means (107) to collect the probe beam (E) after interaction with the tissue (18) to from an image on the primary detector (19) of the area where the probe beam (E) exits the tissue (18).

8. Apparatus according to Claim 7, wherein the optical means (17) comprises an optical fiber.

9. Apparatus according to Claim 7, wherein the optical fiber is bifurcated at a proximal end with a first plurality of fibers (104) for coupling the beam to the reference detector (29) and a second plurality of fibers (103) for coupling the beam to the tissue (18) for interaction and is bifurcated at a distal end with a third plurality of fibers (107) for coupling the interacted beam to the primary detector (19).

10. Non-invasive apparatus for measuring the concentration of predetermined analytes in living body tissue (18), comprising;
a) transmission means for generating a combined beam (D) of electromagnetic radiation comprised of two alternate and repetitive periods of radiation (B, C) having different wavelengths, the two wavelengths having different absorption coefficients for the analyte being sought, at least one of the wavelengths being tunable (C);
b) a primary detector (19) for detecting the combined beam before (D) and after (E) interacting with the tissue (18) and for generating a primary electrical signal proportional to the intensity of the combined beam before (D) and after (E) the interaction;
c) a reference detector (29) for detecting the combined beam (D) and for generating a reference signal proportional to the intensity of the combined beam (D) and wherein the primary electrical signal and the reference signal are substantially equal in magnitude, thus producing a substantially zero alternating component in the primary signal and the reference signal;
d) coupling means for directing the combined beam (D) onto the tissue (18) so that radiation transmitted or reflected by the tissue (18) is coupled to the primary detector (19);
e) control means (25, 39) for controlling the intensity relation between the radiation periods in response to the reference signal and to tune the wavelength of one of the radiation periods (C) to obtain a substantially zero alternating component in the primary signal;
f) change means (60) for changing the ratio of extracellular fluid content to intracellular fluid content in the tissue (18); and
g) display means (28) responsive to the change in the primary signal produced by the primary detector (19) as a result of the fluid ratio change to measure the concentration of the analyte in the tissue (18); and, optionally, either:
(A) where the combined beam (D, E) passes through the tissue (18) before being detected by the primary detector (19); or
(B) apparatus wherein the change means (60) comprises a mechanism for changing the thickness of the tissue (18).

## Patentansprüche

1. Nicht-invasives Verfahren zur Messung der Konzentration von vorbestimmten Analyten im Gewebe (18) lebender Körper, das die Schritte umfaßt:
a) Erzeugen eines kombinierten Strahls (D) von elektromagnetischer Strahlung, die aus zwei alternierenden und sich wiederholenden Perioden von Strahlung (B, C) mit verschiedenen Wellenlängen besteht, wobei die beiden Wellenlängen verschiedene Absorptionskoeffizienten für den gesuchten Analyten aufweisen, wobei wenigstens eine der Wellenlängen (C) abstimmbar ist;
b) Erfassen des kombinierten Strahls (D) mit einem Primärdetektor (19) zum Erzeugen eines primären elektrischen Signals proportional zur Intensität des kombinierten Strahls (D);
c) Erfassen des kombinierten Strahls (D) mit einem Bezugsdetektor (29) zum Erzeugen eines Bezugssignals proportional zur Intensität des kombinierten Strahls (D) und worin die von den Strahlungen mit zwei Perioden in beiden Detektoren (19, 29) erzeugte elektrische Antwort in ihrer Größe im wesentlichen gleich ist, wodurch eine Wechselkomponente von im wesentlichen Null im primären elektrischen Signal und dem Bezugssignal erzeugt wird;
d) Richten des kombinierten Strahls (D) auf das Gewebe (18), so daß vom Gewebe (18) durchgelassene oder reflektierte Strahlung den Primärdetektor (19) erreicht,
e) Steuern des Intensitätsverhältnisses zwischen den Perioden unter Verwendung des Bezugssignals und Abstimmen der Wellenlänge (C) einer der Perioden, um eine Wechselkomponente von im wesentlichen Null im primären Signal zu erhalten;
f) Ändern des Verhältnisses des Gehalts an extrazellulärem Fluid zum Gehalt an intrazellulärem Fluid im Gewebe (18); und
g) Messen der Konzentration des Analyten im Gewebe (18) auf der Basis der Änderung im primären elektrischen Signal als Folge der Änderung des Fluidverhältnisses.

2. Verfahren nach Anspruch 1, bei dem der kombinierte Strahl (D) durch das Gewebe (18) hindurchgeht, bevor er vom Primärdetektor (19) erfaßt wird; und worin gegebenenfalls der Schritt des Änderns des Fluidverhältnisses das Ändern der Dicke des Gewebes (18) umfaßt, zum Beispiel worin entweder die Änderung der Dicke des Gewebes (18) im wesentlichen gleich dem Inversen des Extinktionskoeffizienten des Gewebes (18) bei den beiden Wellenlängen ist oder worin die Änderung der Dicke des Gewebes (18) zyklisch wiederholt wird und die Änderung des primären elektrischen Signals über den Zyklus als Maß für die Konzentration des Analyten im Blut verwendet wird.

3. Verfahren nach Anspruch 1, worin entweder:
(a) der Schritt des Änderns des Fluidverhältnisses das Anwenden von Druck auf die Oberfläche des Gewebes (18) beinhaltet; oder
(b) die Änderung des Fluidverhältnisses durch natürliches Pulsieren aufgrund des Herzschlags bewirkt wird und der Schritt des Messens der Konzentration zu solchem Pulsieren synchronisiert wird; oder
(c) der Analyt ein homologes Körpermaterial ist, und die Wellenlängen im Bereich von 1 bis 2,5 µm liegen.

4. Verfahren nach Anspruch 1 zum Messen der Glucosekonzentration in menschlichem oder tierischem Körpergewebe (18) und wobei eine der Wellenlängen aus dem Intervall von 2125-2185 nm und eine weitere Wellenlänge aus dem Intervall von 2240-2300 nm ausgewählt ist.

5. Verfahren nach Anspruch 1 und entweder:
(a) worin der Analyt Glucose ist und eine der Wellenlängen aus dem Intervall von 1500-1650 nm ausgewählt ist und eine weitere der Wellenlängen aus dem Intervall von 1650-1800 nm ist; oder
(b) worin der Schritt des Messens der Konzentration unter Verwendung eines oder mehrerer zusätzlicher Wellenlängenpaare wiederholt wird und das gewünschte Ergebnis aus den Signalwerten bei jedem Wellenlängenpaar unter Anwendung einer mathematischen Umformung erhalten wird; oder
(c) das ferner vor dem Schritt des Richtens des kombinierten Strahls (D) das Abgleichen des Primärdetektors (19) und des Bezugsdetektors (29) durch Steuern des Intensitätsverhältnisses der Perioden der verschiedenen Wellenlängen (B, C), um kontinuierlich ein minimales Wechselsignal vom Primärdetektor (19) zu erzeugen, Abstimmen der abstimmbaren Wellenlänge (C) über den Abstimmbereich und gleichzeitiges Aufzeichnen eines Steuersignals, das erforderlich ist, um die Bedingung des minimalen Wechselsignals herzustellen in einem Speicher als Funktion der abgestimmten Wellenlänge (C); oder
(d) das ferner, während das Gewebe (18) im kombinierten Strahl (D) ist, ein Kalibrieren der Antwort des Systems durch Ausblenden einer von einem Paar von Wellenlängeperioden und Messen der sich ergebenden Signalamplitude der Wellenlänge des durchgelassenen Wechselsignals und Verwenden des Ergebnisses zur Normierung der Antwort des Systems umfaßt.

6. Vorrichtung zur nicht-invasiven Messung der Konzentration in vivo eines vorbestimmten Analyts in Körpergewebe (18):
a) eine Strahlungseinrichtung (10) zum Erzeugen eines Prüfstrahls (D) aus elektromagnetischer Strahlung, der zwei alternierende Wellenlängenanteile (B, C) enthält, wo wenigstens eine der Wellenlänge (C) abstimmbar ist und die Intensität der Strahlung während wenigstens eines Wellenlängenabschnitts steuerbar ist;
b) eine optische Einrichtung (17) zum Übertragen des Prüfstrahls zum Gewebe;
c) eine Bezugsdetektoreinrichtung (29) zum Erfassen eines repräsentativen Anteils (F) des Prüfstrahls vor der Wechselwirkung mit dem Gewebe (18) und zum Erzeugen eines Bezugssignals proportional zur Intensität des Prüfstrahls (D);
d) eine Primärdetektoreinrichtung (19) zum Erfassen wenigstens eines Anteils der Strahlung des Prüfstrahls (E) nach der Wechselwirkung mit dem Gewebe (18) und zum Erzeugen eines primären Signals proportional zur Intensität des beeinflußten Prüfstrahls (E);
e) eine elektrische Einrichtung (34) zum Erzeugen eines Ausgleichssignals in Antwort auf das primäre Signal;
f) eine elektrische Einrichtung (34) zum Erzeugen eines Steuersignals aus dem Bezugssignal und dem Ausgleichssignal;
g) eine Steuereinrichtung (39) zum Steuern des Intensitätsverhältnisses der alternierenden Anteile (B, C) des Prüfstrahls entsprechend dem Steuersignal;
h) eine Änderungseinrichtung (60) zum Ändern des Verhältnisses des Gehalts an extrazellulärem Fluid zum Gehalt an intrazellulärem Fluid im Gewebe (18); und
i) eine Recheneinrichtung (22) zum Umwandeln der Änderung im primären Signal als Folge der Änderung des Fluidverhältnisses in Konzentrationswerte des (der) gesuchten Analyts (Analyten).

7. Vorrichtung nach Anspruch 6, worin die optische Einrichtung (17) eine Einrichtung (107) zum Sammeln des Prüfstrahls (E) nach der Wechselwirkung mit dem Gewebe (18) beinhaltet, um am Primärdetektor (19) ein Bild des Bereichs zu bilden, wo der Prüfstrahl (E) das Gewebe (18) verläßt.

8. Vorrichtung nach Anspruch 7, worin die optische Einrichtung (17) einen Lichtwellenleiter umfaßt.

9. Vorrichtung nach Anspruch 7, worin der Lichtwellenleiter an einem proximalen Ende mit einer ersten Vielzahl von Fasern (104) zum Verbinden des Strahls mit dem Bezugsdetektor (29) und einer zweiten Vielzahl von Fasern (103) zum Verbinden des Strahls zum Gewebe (18) zur Wechselwirkung gegabelt ist und an einem distalen Ende mit einer dritten Vielzahl von Fasern (107) zum Verbinden des beeinflußten Strahls mit dem Primärdetektor (19) gegabelt ist.

10. Nicht-invasive Vorrichtung zum Messen der Konzentration vorbestimmter Analyten in lebendem Körpergewebe (18), welche umfaßt:
a) eine Übertragungseinrichtung zum Erzeugen eines kombinierten Strahls (D) aus elektromagnetischer Strahlung, die aus zwei alternierenden und sich wiederholenden Perioden von Strahlung (B, C) mit verschiedenen Wellenlängen besteht, wobei die beiden Wellenlängen verschiedene Absorptionskoeffizienten für den gesuchten Analyten aufweisen, wobei wenigstens eine der Wellenlänge abstimmbar (C) ist;
b) einen Primärdetektor (19) zum Erfassen des kombinierten Strahls vor (D) und nach (E) in Wechselwirkung mit dem Gewebe (18) und zum Erzeugen eines primären elektrischen Signals proportional zur Intensität des kombinierten Strahls vor (D) und nach (E) der Wechselwirkung;
c) einen Bezugsdetektor (29) zum Erfassen des kombinierten Strahls (D) und zum Erzeugen eines Bezugssignals proportional zur Intensität des kombinierten Strahls (D) und worin das primäre elektrische Signal und das Bezugssignal in ihrer Größe im wesentlichen gleich sind, wodurch eine Wechselkomponente von im wesentlichen Null im primären Signal und dem Bezugssignal erzeugt wird;
d) eine Verbindungseinrichtung zum Richten des kombinierten Strahls (D) auf das Gewebe (18), so daß vom Gewebe (18) durchgelassene oder reflektierte Strahlung mit dem Primärdetektor (19) verbunden wird;
e) Steuermittel (25, 39) zum Steuern des Intensitätsverhältnisses zwischen den Strahlungsperioden in Reaktion auf das Bezugssignal und zum Abstimmen der Wellenlänge einer der Strahlungsperioden (C), um eine Wechselkomponente von im wesentlichen Null im primären Signal zu erhalten;
f) eine Änderungseinrichtung (60) zum Ändern des Verhältnisses des Gehalts an extrazellulärem Fluid zum Gehalt an intrazellulärem Fluid im Gewebe (18); und
g) eine Anzeigeeinrichtung (28), die auf die vom Primärdetektor (19) als Folge der Änderung des Fluidverhältnisses erzeugte Änderung im primären Signal reagiert, um die Konzentration des Analyts im Gewebe (18) zu messen; und gegebenenfalls entweder:
(A) der kombinierte Strahl (D, E) durch das Gewebe (18) hindurchgeht, bevor er vom Primärdetektor (19) erfaßt wird; oder
(B) eine Vorrichtung, worin die Änderungseinrichtung (60) eine Einrichtung zum Ändern der Dicke des Gewebes (18) umfaßt.

## Revendications

1. Procédé sans ingérence pour mesurer la concentration d'analytes prédéterminés dans le tissu (18) de corps vivants, comprenant les étapes consistant à :
a) produire un faisceau combiné (D) de rayonnement électromagnétique comprenant deux périodes alternées et répétitives de rayonnement (B, C) ayant des longueurs d'onde différentes, les deux longueurs d'onde ayant des coefficients d'absorption différents pour l'analyte qui est recherché, au moins l'une des longueurs d'onde (C) étant accordable;
b) détecter le faisceau combiné (D) avec un détecteur primaire (19) pour produire un signal électrique primaire proportionnel à l'intensité du faisceau combiné (D);
c) détecter le faisceau combiné (D) avec un détecteur de référence (29) pour produire un signal de référence proportionnel à l'intensité du faisceau combiné (D) et dans lequel la réponse électrique produite par les rayonnements des deux périodes dans les deux détecteurs (19,29) est sensiblement égale en amplitude, ce qui conduit à une composante alternative sensiblement nulle dans le signal électrique primaire et dans le signal de référence;
d) diriger le faisceau combiné (D) sur le tissu (18) de sorte qu'un rayonnement transmis ou réfléchi par le tissu (18) atteint le détecteur primaire (19);
e) commander la relation d'intensité entre les périodes en utilisant le signal de référence et accorder la longueur d'onde (C) de l'une des périodes pour obtenir une composante alternative sensiblement nulle dans le signal primaire;
f) modifier le rapport du contenu en fluide extra-cellulaire au contenu en fluide intra-cellulaire dans le tissu (18);
g) mesurer la concentration de l'analyte dans le tissu (18) sur la base de la variation du signal électrique primaire en tant que résultat de la variation du rapport des fluides.

2. Procédé selon la revendication 1, dans lequel le faisceau combiné (D) traverse le tissu (18) avant d'être détecté par le détecteur primaire (19); et facultativement, dans lequel l'étape de modification du rapport des fluides consiste à modifier l'épaisseur du tissu (18), par exemple soit par le fait que la variation de l'épaisseur du tissu (18) est sensiblement égale à l'inverse du coefficient d'extinction du tissu (18) pour les deux longueurs d'onde, soit par le fait que la variation de l'épaisseur du tissu (18) est répétée cycliquement, et que la variation du signal électrique primaire pendant le cycle est utilisée en tant que mesure de la concentration de l'analyte dans le sang.

3. Procédé selon la revendication 1, selon lequel :
(a) l'étape de modification du rapport des fluides inclut l'application d'une pression à la surface du tissu (18); ou
(b) la modification du rapport des fluides est provoquée par une pulsation naturelle due au battement cardiaque, et l'étape de mesure de la concentration est synchronisée sur une telle pulsation; ou
(c) l'analyte est une substance homologue du corps et les longueurs d'onde se situent dans la gamme de 1 à 2,5 microns.

4. Procédé selon la revendication 1 pour mesurer la concentration du glucose dans le tissu (18) d'un corps humain ou d'un corps animal et comprenant l'une des longueurs d'onde sélectionnées pour l'intervalle 2125-2185 nm et l'autre des longueurs d'onde pour l'intervalle 2240-2300 nm.

5. Procédé selon la revendication 1 et :
(a) selon lequel l'analyte est du glucose et l'une des longueurs d'onde est sélectionnée dans l'intervalle 1500-1650 nm et une autre des longueurs d'onde est sélectionnée dans l'intervalle 1650-1800 nm; ou
(b) dans lequel l'étape de mesure de la concentration est répétée en utilisant un ou plusieurs couples additionnels de longueurs d'onde et le résultat désiré est obtenu à partir des valeurs des signaux pour chaque couple de longueurs d'onde en utilisant une transformation mathématique; ou
(c) comportant en outre, avant l'étape consistant à diriger le faisceau combiné (D), l'égalisation du détecteur primaire (19) et du détecteur de référence (29) par commande de la relation entre les intensités des périodes des différentes longueurs d'onde (B, C) de manière à produire continûment un signal alternatif minimum à partir du détecteur primaire (19), le réglage d'accord de la longueur d'onde accordable (C) dans la gamme de réglage d'accord et simultanément l'enregistrement dans une mémoire, en fonction de la longueur d'onde accordée (C), d'un signal de commande requis pour produire la condition du signal alternatif minimum; ou
(d) comprenant en outre, alors que le tissu (18) est dans le faisceau combiné (D), l'étalonnage de la réponse du système par annulation d'une période d'un couple de périodes de longueurs d'onde et la mesure de l'amplitude résultante transmise du signal à longueurs d'onde alternatives et l'utilisation du résultat pour normaliser la réponse du système.

6. Dispositif pour une mesure sans ingérence de la concentration in vivo d'un analyte prédéterminé dans le tissu (18) d'un corps, comprenant
a) des moyens de rayonnement (10) pour produire un faisceau sonde de rayonnement électromagnétique (D) contenant deux parties de longueurs d'onde alternatives (B, C), au moins l'une des longueurs d'onde (C) étant accordable et l'intensité du rayonnement pendant au moins une partie des longueurs d'onde est commandable;
b) des moyens optiques (17) pour appliquer le faisceau sonde au tissu;
c) des moyens (29) formant détecteur de référence pour détecter une partie représentative (F) du faisceau sonde, avant son interaction avec le tissu (18), et pour produire un signal de référence proportionnel à l'intensité du faisceau sonde (D);
d) des moyens (19) formant détecteur primaire pour détecter au moins une partie du rayonnement (E) du faisceau sonde après son interaction avec le tissu (18) et produire un signal primaire proportionnel à l'intensité du faisceau sonde (E) qui a agit sur le tissu;
e) des moyens électriques (34) pour produire un signal de décalage en réponse au signal primaire;
f) des moyens électriques (35) pour produire un signal de commande à partir du signal de référence et du signal de décalage;
g) des moyens de commande (39) pour commander la relation d'intensité des parties alternatives (B, C) des faisceaux sondes, en fonction du signal de commande;
h) des moyens de modification (60) pour modifier le rapport du contenu- du fluide extra-cellulaire au contenu du fluide intra-cellulaire dans le tissu (18); et
i) des moyens de calcul (22) pour convertir la variation du signal primaire en tant que résultat de la variation du rapport des fluides, en des valeurs de concentration du ou des analytes recherchés.

7. Dispositif selon la revendication 6, dans lequel les moyens optiques (17) comprennent des moyens (107) pour collecter le faisceau sonde (E) après son interaction avec le tissu (18) pour former, sur le détecteur primaire (19) une image de la zone dans laquelle le faisceau sonde (E) sort du tissu (18).

8. Dispositif selon la revendication 7, dans lequel les moyens optiques (17) comprennent une fibre optique.

9. Dispositif selon la revendication 7, dans lequel une fibre optique est bifurquée sur une extrémité proximale et comprend une première pluralité de fibres (104) pour coupler le faisceau au détecteur de référence (29) et une seconde pluralité de fibres (103) pour coupler le faisceau au tissu (18) pour l'interaction, et est bifurquée au niveau d'une extrémité distale avec une troisième pluralité de fibres (107) pour coupler le faisceau d'interaction au détecteur primaire (19).

10. Dispositif sans ingérence pour la mesure de la concentration d'analytes prédéterminé dans le tissu (18) d'un corps vivant, comprenant :
a) des moyens de transmission pour produire un faisceau combiné (D) de rayonnement électromagnétique constitué de deux périodes alternatives et répétitives de rayonnement (B, C) ayant des longueurs d'onde différentes, les deux longueurs d'onde ayant des coefficients d'absorption différents pour l'analyte recherché, au moins l'une des longueurs d'onde étant accordable (C);
b) un détecteur primaire (19) pour détecter le faisceau combiné avant (D) et après (E) son interaction avec le tissu (18) et pour produire un signal électrique primaire proportionnel à l'intensité du faisceau combiné avant (D) et après (E) l'interaction;
c) un détecteur de référence (29) pour détecter le faisceau combiné (D) et pour produire un signal de référence proportionnel à l'intensité du faisceau combiné (D) et dans lequel le signal électrique primaire et le signal de référence possèdent des amplitudes sensiblement égales, ce qui produit une composante alternative sensiblement nulle dans le signal électrique primaire et dans le signal de référence;
d) des moyens de couplage pour diriger le faisceau combiné (D) dans le tissu (18) de sorte que le rayonnement transmis ou réfléchi par le tissu (18) est couplé au détecteur primaire (19);
e) des moyens de commande (25,39) pour commander la relation d'intensité entre les périodes de rayonnement en réponse au signal de référence et accorder la longueur d'onde d'une des périodes de rayonnement (C) pour obtenir une composante alternative sensiblement nulle dans le signal primaire;
f) des moyens de modification (60) pour modifier le rapport du contenu en fluide extra-cellulaire au contenu en fluide intra-cellulaire dans le tissu (18); et
g) des moyens d'affichage (28) aptes à répondre à la variation du signal primaire produit par le détecteur primaire (19) en tant que résultat du rapport de la variation du rapport des fluides pour mesurer la concentration de l'analyte dans le tissu (18); et facultativement :
(A) dans le cas où le faisceau combiné (D, E) traverse le tissu (18) devant être détecté par le détecteur primaire (19); ou
(B) dispositif dans lequel les moyens de modification (60) comprennent un mécanisme pour modifier l'épaisseur du tissu (18).
